(19) Europäisches Patentamt
European Patent Office
Office européen des brevets

(11) EP 1 776 986 A2

(12) # EUROPEAN PATENT APPLICATION

(43) Date of publication:
**25.04.2007 Bulletin 2007/17**

(21) Application number: **06255439.9**

(22) Date of filing: **23.10.2006**

(51) Int Cl.:
***A61Q 17/04*** *(2006.01)*
***A61K 8/25*** *(2006.01)*
***A61K 8/27*** *(2006.01)*
***A61K 8/29*** *(2006.01)*
***A61K 8/81*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **24.10.2005 US 257297**

(71) Applicant: **Johnson and Johnson Consumer Companies, Inc.**
**Skillman, NJ 08558 (US)**

(72) Inventors:
- **LiBrizzi, Joseph J.**
**Hillsborough, NJ 08844 (US)**
- **Cossa, Anthony J.**
**Branchburg, NJ 08876 (US)**
- **Edwards, Elizabeth**
**Lawrenceville, NJ 08648 (US)**
- **Bussey, Deborah**
**Hamilton, NJ 08690 (US)**

(74) Representative: **Mercer, Christopher Paul et al**
**Carpmaels & Ransford**
**43-45 Bloomsbury Square**
**London WC1A 2RA (GB)**

(54) **Sunscreen compositions comprising polymeric emulsifiers and methods of using the same**

(57) Provided are oil-in-water sunscreen compositions comprising inorganic sunscreens and polymeric emulsifiers that are substantially free of hydrophobic modification, and methods of using the same.

EP 1 776 986 A2

## Description

FIELD OF THE INVENTION

The present invention relates to oil-in-water emulsions comprising polymeric emulsifiers and, more particularly, to oil-in-water emulsions comprising polymeric emulsifiers and sunscreen agents that exhibit unique combinations of sun-protection, rinse-resistance, and/or low-irritation properties.

BACKGROUND

It is well documented that human skin is sensitive to sunlight and artificial light containing radiation of wavelengths between about 290 nanometers (nm) and 400 nm. Light radiation with wavelengths of between 290 nm and 320 nm, which are known as UV-B rays, tend to cause skin bums and erythema that can harm the development of a natural tan. UV-A rays, with wavelengths of between 320 nm and 400 nm, which tend to cause tanning of the skin, are also liable to induce an impairment in the skin, especially in the case of sensitive skin or skin that is continually exposed to solar radiation. UV-A rays in particular tend to bring about a loss of elasticity of the skin and the appearance of wrinkles, leading to premature aging. They tend to promote the triggering of the erythmal reaction or amplify this reaction in certain individuals and may even be the cause of phototoxic or photoallergic reactions. Applicants have thus recognized that it is desirable to screen out UV-A and UV-B radiations from the skin, for example, by applying a topical composition to the skin which tends to screen such harmful radiation therefrom and prevent photodamage.

Applicants have recognized that it is highly desirable for topical sunscreen compostions to exhibit not only effective and high performance in screening UV radiation from the skin, but also, to exhibit good aesthetics (e.g., non-greasy feel and non-whitening of the skin) and/or relatively low irritation to the skin and eyes. Reducing irritation and sensitization of sunscreen products is especially important when considering the use of these products on infants and young children. Furthermore, it is also desirable in many instances for the sunscreen to resist washing away from water and perspiration, so that frequent reapplication is unnecessary. Applicants have recognized that conventional sunscreen compositions tend not to achieve such desirable combinations of properties.

For example, certain conventional sunscreen compositions have employed the use of inorganic sunscreen agents, such as titanium dioxide and zinc oxide, in an attempt to enhance mildness of the sunscreen formulation. However, many of such inorganic sunscreen formulations, while reducing irritation and sensitization, suffer from aesthetic issues such as whitening of skin. Additionally, to provide a required SPF and good phase stability, inorganic sunscreen agents are typically formulated as water-in-oil emulsions, which are not preferred for aesthetic reasons.

Other attempts to formulate sunscreen compositions have been made, including for example, products described in U.S. Pat. Nos. 5,980,871, and 6,540,986 B2 to Lukenbach et al. (directed to inorganic sunscreen systems containing an inorganic sunscreen agent; an anionic emulsifier, an oil component and at least one emollient), U.S. Pat. Appl. No. 2004/0067208 A1 to Lennon et al. (directed to the use of a polyolefin-derived amphiphilic oligomer or polymer, containing at least one polar moiety and an effective amount of a metal oxide UV-blocking agent), and U.S. Pat.Appl. No. 2004/0071641A1 to Boutelet et al. (directed to the use of an amphiphilic polymer having at least one ethylenically unsaturated monomer which comproses a sulfonic group and contains at least one hydrophobic moiety and an insoluble mineral and/or organic sunscreen). However, applicants have recognized that while such systems tend to produce compositions having sufficient SPF for certain uses, they also tend to lack one or more of the rinse-resistance, aesthetics, and/or low-irritation properties desirable in sunscreen compositions.

Thus, there still remains a need to provide an effective sun protection from UVA and UVB rays, that is resistant to washing away from water and perspiration, is easy and convenient to apply, maintains invisibility (non-whitening), is non-staining, non-greasy and possesses low irritation to skin and eyes.

SUMMARY OF INVENTION

Applicants have discovered unexpectedly that inorganic sunscreens can be combined with certain polymeric emulsifiers to achieve sunscreen compositions that exhibit a desirable and surprising combination of sun protection, rinse-resistance, and low-irritation properties as compared to conventional sunscreens. Accordingly, one aspect of the present invention relates to oil-in-water sunscreen compositions comprising an inorganic sunscreen, a polymeric emulsifier that is substantially free of hydrophobic modification, which sunscreen composition is substantially free of monomeric emulsifiers.

Another aspect of the present invention relates to oil-in-water sunscreen compositions comprising an inorganic sunscreen, a polymeric emulsifier that is substantially free of hydrophobic modification, which sunscreen composition is substantially free of organic sunscreens.

Applicants have further discovered that certain compositions comprising an inorganic sunscreen comprising

both titanium dioxide and zinc oxide have not only desirable SPF, aesthetics, and mildness, but also surprising rinse resistance properties associated therewith. Thus, another aspect of the present invention relates to oil-in-water sunscreen compositions comprising an inorganic sunscreen comprising a mixture of titanium dioxide and zinc oxide, and a polymeric emulsifier that is substantially free of hydrophobic modification.

Another aspect of the present invention relates to methods of protecting the skin from photodamage comprising applying a composition of the present invention to the skin.

DESCRIPTION OF PREFERRED EMBODIMENTS

Applicants have discovered unexpectedly that hydrophobically-modified polymeric emulsifiers that are substantially free of hydrophobic modification can be combined with inorganic sunscreen agents, to form compositions that overcome one or more of the disadvantages associated with conventional sunscreen compositions. That is, applicants have recognized that the emulsion compositions of the present invention tend to exhibit relatively high sun protection factor (SPF), as well as relatively high rinse-resistance, favorable aesthetics, low skin irritation, and/or low ocular irritation as compared to conventional emulsion compositions.

In particular, applicants have tested the sun protection factor (SPF) associated with compositions of the present invention via the "SPF Test", described in detail below, wherein, as recognized by those of skill in the art, a higher SPF value represents a composition that desirably tends to better protect the skin from harmful effects of ultraviolet radiation. Applicants have discovered unexpectedly that the compositions of the present invention exhibit an SPF value that is unexpectedly high as compared to conventional comparable compositions. For example, in certain embodiments, the present compositions exhibit an SPF value that is at least about 10 or higher, preferably at least about 12 or higher, more preferably at least about 15 or higher, and even more preferably at least about 20 or higher. In certain more preferred embodiments, the present compositions exhibit an SPF value that is at least about 25 or higher, preferably at least about 30 or higher, more preferably at least about 45 or higher, and more preferably about 60 or higher. In certain preferred embodiments, such SPF values tend to be at least about 2 times, preferably at least about about 3 times, more preferably at least about 4 times or more greater than the SPF values associated with comparable compositions outside of the scope of the present invention.

Applicants have further measured the rinse resistance properties of compositions of the claimed invention using the Rinse Resistance Measurement described in detail below wherein the percentage of initial SPF remaining after immersion in water for 40 minutes (%$R_{40}$) and 80 minutes (%$R_{80}$) is measured and wherein, as will be recognized by those of skill in the art, a higher percentage (%$R_{40}$/%$R_{80}$) correlates to a desirably higher rinse resistance of the composition. Applicants have discovered that the present compositions tend to exhibit surprisingly high %$R_{40}$ and %$R_{80}$ values as compared to other sunscreen compositions. For example, in certain embodiments, the present compositions exhibit a %$R_{40}$ of at least about 80%, preferably at least about 85%, more preferably at least about 90%, more preferably at least about 95%, more preferably at least about 98%, and even more preferably about 100%. Additionally, in certain embodiments, the present compositions exhibit a %$R_{80}$ of at least about 80%, preferably at least about 85%, more preferably at least about 90%, more preferably at least about 90%, more preferably at least about 95%, and more preferably at least about 98%. In certain preferred embodiments, such percentages tend to be at least about 1.2 times, preferably at least about 1.4 times, more preferably about 1.6 times, and more preferably about 2 times or more, greater than the percentages (%$R_{40}$/%$R_{80}$) associated with comparable compositions outside of the scope of the present invention.

Furthermore, applicants have measured the irritation associated with the compositions of the present invention via the Ocular Sting Test described hereinbelow. Applicants have discovered that the present compositions tend to be relatively mild to the eye, and preferably, significantly less irritating to the eye than comparable compositions.

In particularly preferred embodiments, applicants have discovered that oil-in-water sunscreen compositions having an SPF of at least 10, preferably at least 15, an %$R_{40}$ value of at least about 90%, preferably about 95%, an %$R_{80}$ value of at least about 90%, preferably about 95%, that is further mild to the eyes can be achieved by combining and inorganic sunscreen and a polymeric emulsifier in accord with the present invention.

Any of a variety of suitable inorganic sunscreen agents may be used in the compositions of the present invention. Applicants have recognized that for certain embodiments, the inorganic sunscreen agents are preferably selected to be dispersible in water having a particle diameter of from about 0.01 μm to about 500 μm, preferably from about 0.1 μm to about 500 μm, more preferably from about 0.5 μm to about 300 μm, more preferably from 1.0 μm to about 200 μm, and more preferably from about 1 μm to about 50 μm and preferably active in the UV-A and/or UV-B regions (absorbers). In certain preferred embodiments, the inorganic sunscreen agents comprise microfine particles having a diameter of less than 40 microns, preferably less than 1 micron, more preferably from about 0.1 to about 0.5 microns. In certain embodiments, the inorganic sunscreen agents may be present with or without surface coatings comprising one or more hydrophobic moieties. (e.g., silicone compounds and aluminum stearate, coatings of fatty acids, fatty esters, hydrophobic surface modification such as from certain organosilanes, and the like) that tend to cancel the hydrophilic nature of the

inorganic sunscreen agents. Nonlimiting examples of inorganic sunscreen agents suitable for use herein include metal oxides such as iron oxide, titanium dioxide, zirconium oxide, zinc oxide, transition metal oxides or other oxide pigments, various forms of silica, aluminosilicates, carbonates, combinations of two or more thereof, and the like. For certain preferred embodiments, titanium dioxide, zinc oxide and combinations thereof are particularly notable.

Applicants have recognized that in certain embodiments, combinations of titanium dioxide and zinc oxide can be used in accord with the present invention to achieve compositions having unexpectedly high rinse resistant properties. Accordingly, certain particularly preferred inorganic sunscreens of the present invention comprise a combination of titanium dioxide and zinc oxide.

Any suitable amount of inorganic sunscreen agents may be used in the compositions of the present invention. In certain embodiments, the present compositions comprise from about 0.01 percent to about 50 percent, preferably from about 0.1 percent to about 35 percent, and more preferably, from about 0.1 percent to about 20 percent by weight of one or more of an inorganic sunscreen agents. As used herein and throughout the application, all weight percents represent percent by weight of active based on the total weight of composition, unless otherwise indicated.

Any of a variety of suitable polymeric emulsifiers that are substantially free of hydrophobic modification may be used according to the present invention. As used herein the term "substantially free of hydrophobic modification" refers generally to polymeric emulsifiers that comprise less than 1 weight %, preferably less than 0.5 weight %, more preferably less than 0.1 weight %, more preferably less than 0.01 weight %, and more preferably less than 0.41 weight % mole percent of hydrophobic moieties comprising greater than four carbon atoms covalently bonded together. As used herein, mole percent is the moles of hydrophobic moieties comprising greater than four carbon atoms covalently bonded together in a polymer divided by the total moles of monomer units of the polymer multiplied by one hundred.

In certain preferred embodiments, applicants have recognized it is desirable to use one or more polymeric emulsifiers that are substantially free of hydrophobic modification, which tends to contribute toward formation of a stable emulsion between and oil and aqueous phase, provide shelf stability to the composition into which it is added and/or enhance the SPF of the composition. In addition, certain preferred polymeric emulsifiers comprise those compounds that are water-soluble and are capable of forming a phase stable emulsion, preferably stable for at least about 1 week, more preferably at least about a month, of a mineral oil in water. (As used herein a material is defined as "water-soluble", if it is possible to form a clear solution by adding only 0.5% by weight of the material in deionized water that is stable at room temperature (no settling or phase-instability) for 48 hours.) Certain preferred polymeric emulsifiers of the present invention are salt-sensitive, in that, their solubility in water is reduced, often dramatically, in the presence of electrolytes (such as electrolytes typically present on the surface of skin). A polymeric emulsifier is defined as "salt-sensitive" if it loses its ability to remain phase stable in aqueous solution when sodium chloride has been added. Specifically, a "salt sensitive" polymeric emulsifier will show phase separation and/or a 30% or more change in viscosity (measured using a Brookfield viscometer with an LVT2 spindle at 12 RPM) if 3% sodium chloride is added to a homogenous solution of 1% (active) polymeric emulsifier in deionized water.

The polymeric emulsifiers in the present invention may be of any suitable molecular weight. In certain embodiments of the invention, the polymeric emulsifier has a weight average molecular weight that is preferably greater than about 100,000, more preferably greater than about 250,000, and even more preferably greater than about 500,000.

The polymeric emulsifier of the present invention may comprise one or more monomers (i.e., the polymeric emulsifier may be a homopolymer, a copolymer, a terpolymer, etc.). In embodiments wherein the polymer comprises two or more monomers polymerized together, any suitable ratio of such two or more monomers may be used. In one preferred embodiment, the polymeric emulsifiers may be capable of being represented by the following chemical formula;

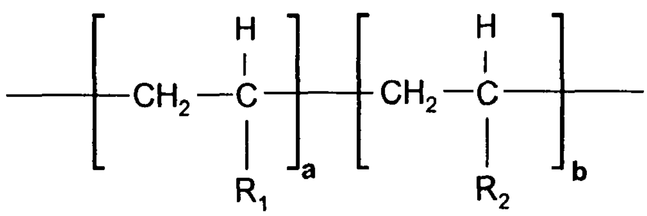

wherein a is 0 to 0.99, b is 0.01 to 1, a+b=1, $R_1$ and $R_2$ are either identical or different moieties and are both free of linear, branched, or cyclic moieties having more than four covalently linked carbon atoms.

In one preferred embodiment, $R_1$ can be represented by

$X\text{-}Y\text{-}Z^- M^+$;

wherein X is a moiety selected from the group of-COO-, -OCO-, -CONH-and-NHCO-, Y is a linear or branched or cyclic ($C_{1-4}$) alkyl group, $Z^-$ is an anionic functional group such as $-COO^-$, $-SO_3^-$, $-SO_4^-$, $-PO_3^-$ or $-P0_4^-$, and $M^+$ is an alkali metal such as $Li^+$, $Na^+$ or $K^+$ or an ammonium counterion as $NH_4^+$.

In one preferred embodiment of the present invention, X is NHCO-, Z is $-SO_3^-$, and Y is a methyl group, i.e., $R^1$ is an acrylamidoalkylsulfonate (AMPS) moiety.

In another preferred embodiment, $R_2$ can be represented by

$$X\text{-}Y\text{-}Z^- M^+;$$

wherein X, Y, Z, and M may be selected from any of those moieties as defined above for $R_1$.

In certain other preferred embodiments, $R_2$ represents a nonionic linear or branched or cyclic group that includes a chain of 0 to 4 covalently bonded carbon atoms. For example, $R_2$ may include a cyclic moiety, such as a heterocycle that includes a carbon, nitrogen, oxygen, or combinations thereof.

In certain other preferred embodiment of the present invention, $R_1$ is an AMPS moiety and $R_2$ represents a nonionic linear or branched or cyclic group that includes 0 to 4 heteroatoms selected from nitrogen or oxygen.

The polymeric emulsifier may exist in varying forms, including random polymers, block, star, graft, and the like. Notable commercially available polymeric emulsifiers that are substantially free of hydrophobic modification include, but are not limited to, a copolymer of acrylamidoalkyl sulfonic acid and cyclic N-vinylcarboxamides, commercially available under the tradename Aristoflex® AVC by Clariant, AMPS homopolymers, such as a homopolymer of acrylamidoalkyl sulfonic acid commercially available under the tradename Granthix APP by Grant Industries, Inc. which is supplied as a mixture of ammonium polyacryloyldimethyltaurate with isohexadecane and polysorbate 80.

Any suitable amounts of polymeric emulsifiers that are substantially free of hydrophobic modification may be used in the compositions of the present invention. In certain preferred embodiments, the compositions of the present invention comprise at least about 0.3 weight percent, of polymeric emulsifier. In certain more preferred embodiments, the present compositions comprise, for example, between about 0.3% and about 3%, between about 0.3% and about 2%, and between about 0.3% and about 1% of such polymeric emulsifiers.

According to certain embodiments, the present compositions further comprise one or more wetting agents. As used herein, the term "wetting agent" refers to anionic surfactants possessing at least one sulfonate group (moiety) and at least one base-neutralizable carboxylic acid group (moiety) that are suitable for facilitating the wetting of a substrate with a composition of the present invention and allowing the hydrophobic agent of the composition to tend to remain on the substrate (for example by avoiding re-emulsification of the hydrophobic agent) as a wetting agent. Non-limiting examples of suitable wetting agents include sulfosuccinates, such as, disodium laureth sulfosuccinate, dioctyl sodium sulfosuccinate, sodium methyl 2-sulfolaurate, and the like, isethiones, such as, sodium cocoyl isethionate, and the like, and sulfoacetates, such as, sodium lauryl sulfoacetate, and the like. A variety of such wetting agents are available commercially from various sources including McIntyre Group Limited (disodium laureth sulfosuccinate sold under the tradename MACKANATE EL, dioctyl sodium sulfosuccinate sold under the tradename MACKANATE DOS 70), Stepan Company (sodium methyl 2-sulfolaurate sold under the tradename ALPHA STEP PC 48, sodium lauryl sulfoacetate sold under the tradename LANTHANOL LAL), and Clariant Corporation (sodium cocoyl isethionate sold under the tradename HOSTAPON SCI 85).

Any suitable amount of wetting agent may be used in the compositions of the present invention. In certain embodiments, the present compositions comprise from greater than zero to about one weight percent of wetting agent, preferably from about 0.1 to about one, more preferably from about 0.1 to about 0.5 and even more preferably from about 0.3 to about 0.5 weight percent of active wetting agent.

In certain preferred embodiments, the compositions of the present invention are substantially free of monomeric emulsifiers. As used herein, the term "substantially free of monomeric emulsifiers" refers to a composition comprising less than about 1.0%, preferably less than about 0.5 percent, more preferably less than about 0.1 percent, and even more preferably than about 0.01 percent, or less than about 0.001 percent of monomeric emulsifiers. The term "monomeric surfactant emulsifiers" refers to any one or more materials that are non-polymerized surfactants that can be used to emulsify oil to form an oil-in-water emulsion (water insoluble), other than those falling under the definition of "wetting agent" above. Said monomeric surfactants can be of the anionic, amphoteric cationic or nonionic class of surfactants.

In certain preferred embodiments, the compositions of the present invention are substantially free of organic sunscreens. As used herein, the term "substantially free of organic sunscreens" refers to a composition comprising less than about 1.0%, preferably less than about 0.5 percent, more preferably less than about 0.1 percent, and even more preferably than about 0.01 percent, or less than about 0.001 percent of organic sunscreens, such as those selected from the group consisting of ethylhexyl salicylate, ethylhexyl methoxycinnamate, octocrylene, phenylbenzimidazole sulphonic acid, benzophenone-3, benzophenone4, benzophenone-5, 4-methylbenzylidene camphor, terephthalylidene dicamphor sulphonic acid, disodium phenyl dibenzimidazole tetrasulphonate, 2,4,6-tris(diisobutyl 4'-aminobenzalmalonate)-s-triazine, anisotriazine, ethylhexyl triazonc, diethylhexyl butamido triazone, methylene bisbenzotriazolyl tetramethylbutylphenol, drometrizole trisiloxane, 1,1'-dicarboxy (2,2'-dimethylpropyl)-4,4-diphenylbutadiene, mixtures thereof, and the like.

The compositions of the present invention may further comprise any of a variety of additives or other materials used conventionally. For example, the present compositions may also include dyes, fragrances, and other functional ingredients common to skin care compositions, as long as they do not detract from the phase stability of the personal care composition. In general, in order to maintain phase stability, the level of electrolyte (e.g., ionized moieties other than the wetting agent) is maintained or substantially maintained at a relatively low level, such as less than about 2%, such as less than about 0.5% of the total composition.

The present compositions are preferably formulated to be oil-in-water emulsions that are shelf-stable in that the emulsion does not lose phase stability or "break" when kept at standard conditions (22 degrees Celsius, 50% relative humidity) for a week or more after it is made.

Compositions of the present invention may exhibit any suitable pH for promoting shelf stability and to minimize irritation to the skin and eyes. In one embodiment of the invention, the pH is from about 4.0 to about 9.0, more preferably from about 5.5 to about 7.5. Furthermore, compositions of the present invention may have any suitable viscosity to promote stability of the composition and to promote a pleasant sensation when applied to the skin.

Applicants have recognized that the compositions of the present invention may be used advantageously in a wide variety of applications. For example, in certain preferred embodiments, the present compositions are formulated to be, or be used in, topical, personal care compositions and/or products such as, for example recreational sun care products, everyday moisturizers, color cosmetics as well as other skin care compositions, and the like.

In another embodiment of the invention, the personal care composition is applied to the skin as a moisturizer to reduce the transmission of water vapor there from. In yet another embodiment of the invention, the personal care composition is applied to the face.

In certain preferred embodiments, the personal care compositions of the present invention are preferably applied to the skin and left on the skin for a period of time such as between about 30 mintues and about 24 hours without rinsing with water of soap.

EXAMPLES

<u>EXAMPLES 1-8</u>

The following examples serve as illustrations of the compositions of this invention, however, they do not limit the scope of the invention described herein.

Five personal care compositions, Examples 1, 2, 5, 6 and 8, consistent with embodiments of the invention described herein, were prepared. Three comparative compositions, Comparitive Examples 3, 4, and 7 were also prepared as described herein. Component amounts in this procedure are given in terms of parts by weight per 100 parts of the final personal care composition.

The emulsion systems shown in Examples 1, 2, 5, and 6 were prepared as follows: three (3.0) parts glycerin were added to the amount of parts of water according to Table 1, less 15.0 parts water, solution agitated to uniform, 0.3 parts of parabens added and heated to 60-65°C till the parabens are dissolved. To the solution 0.75 parts of ammonium acryloyldimethyltaurate/ VP copolymer were added and mixed with agitation until the solution was homogeneous, the solution then held between 60-65 °C for phasing (Phase 1 Solution). In a separate container, 7.0 parts of mineral oil and 3.75 parts capric/caprylic triglycerides, were heated to 60-65°C with agitation. In the case of Examples 1 and 2, 4.5 parts titanium dioxide were added and mixed until dispersed. In the case of Examples 5 and 6, 4.0 parts zinc oxide were added and mixed until dispersed and then maintained between 60-65°C temperature. The resulting solution was then added to above solution (Phase 1 Solution) with rapid agitation. Heat was turned off, and 15 parts water added to cool the batch, agitation was continued until solution became homogenous. In the case of Examples 5 and 6, 4.5 parts titanium dioxide was added and the mixture was homogenized. In the case of Examples 2 and 6, 0.65 and 0.10 parts of Sodium Methyl 2-Sulfolaurate and Disodium 2-Sulfolaurate were added respectively and the mixture was homogenized. The mixture was cooled to 35°C, 1.0 part of phenoxyethanol added to Example 6 and agitated until complete dissolution was achieved. In the case of examples 1 and 2, the pH was adjusted if necessary to be within the range of 5.5-6.5. In the case of examples #5 and #6 (examples that included zinc oxide), the pH was adjusted if necessary to be within the range of 7.0-8.0.

The emulsion systems shown in Examples 3 and 4 were prepared as follows:

3.0 parts glycerin were added to the amount of parts of water according to Table 1, less 15.0 parts water, solution agitated to uniform. 0.75 parts of hydroxyethylcellulose was added, and heated with constant stirring to 60-65°C until complete dissolution of hydroxyethylcellulose was achieved, the solution was then held between 60-65°C for phasing (Phase 2 Solution). In a separate container, 7.0 parts of mineral oil and 3.75 parts capric/caprylic triglycerides

were heated to 60-65°C with agitation. 1.56 parts laureth-4, 0.44 parts steareth-21, and 4.0 parts cetyl alcohol were added and mixed until melted and became homogeneous. 4.5 parts titanium dioxide were added and mixed until dispersed, and then maintained between 60-65°C temperature, this solution was then added to above solution (Phase 2 Solution) with rapid agitation. The heat was turned off, and 15.0 parts water added to cool the batch, agitation continued till solution became homogenous. In the case of Example 4, 0.65 parts of Sodium Methyl 2-Sulfolaurate Disodium 2-Sulfolaurate were added and mixed until homogeneous. The mixture was then cooled to 35°C, 0.3 parts of parabens and phenoxyethanol were added and agitated until complete dissolution was achieved. The pH was adjusted if necessary to be within the range of 5.5-6.5.

The emulsion system shown in Example 7 was prepared as follows:

3.0 parts glycerin was added to the amount of parts of water according to Table 1, less 15.0 parts water. The resulting solution was agitated to uniform. 0.30 parts of parabens were added, and heated to 60-65°C till the parabens are dissolved. To such mixture, 0.75 parts of hydroxyethylcellulose were added, and the mixture was stirred until hydroxyethylcellulose dissolves, the solution was then held between 60-65 °C for phasing (Phase 3 solution). In a separate container, 7.0 parts of mineral oil and 3.75 parts capric/caprylic triglycerides, were heated to 60-65°C with agitation. 1.56 parts laureth-4, 0.44 parts steareth-21, and 4.0 parts cetyl alcohol were added and mixed until melted and the mixture became homogeneous. To this mixture 4.0 parts zinc oxide were added and mixed until dispersed, and then maintained between 60-65°C temperature. This solution was then added to the above solution (Phase 3 Solution) with rapid agitation. The heat was turned off, and 4.5 parts of titanium dioxide added and mixed until uniformly dispersed. To this mixture 15.0 parts water were added to cool the batch, mixing was continued until the solution became homogenous. The mixture then cooled to 30-35°C temperature. The pH was adjusted if necessary to be within the range of 7.0-8.0.

The emulsion system shown in Example 8 was prepared as follows:

3.0 parts glycerin were added to the amount of parts of water according to Table 1, less 15.0 parts water, and the solution agitated to uniform. 0.3 parts of parabens were added and heated to 60-65°C until the parabens were dissolved. To this 2.05 parts of Isohexadecane, Ammonium Polyacryloyldimethyltaurate and Polysorbate 80 were added and mixed with agitation until the solution becomes homogeneous. The solution was then held between 60-65 C for phasing (Phase 4 solution). In a separate container, 7.0 parts of mineral Oil and 3.75 parts capric/caprylic triglycerides were heated to 60-65°C with agitation. To this mixture, 4.5 parts titanium dioxide were added and mixed until dispersed and maintained between 60-65°C temperature, and this solution was then added to the above solution (Phase 4 Solution) with rapid agitation. The heat was turned off, and 15 parts water added to cool the batch, and agitation continued until solution became homogenous. The solution was then cooled to 30-35°C temperature. The pH was adjusted if necessary to be within the range of 5.5-6.5.

**TABLE-1**

| INCI Name | Ex.1 | Ex.2 | Comp Ex. 3 | Comp Ex. 4 | Ex.5 | Ex.6 | Comp Ex. 7 | Ex.8 |
|---|---|---|---|---|---|---|---|---|
| Ammonium Acryloyldimethyltaurate/ VP Copolymer | 0.75 | 0.75 | | | 0.75 | 0.75 | | |
| Isohexadecane (and) Ammonium Polyacryloyldimethyltaurate (and) Polysorbate 80 | | | | | | | | 2.05 |
| Glycerin | 3.00 | 3.00 | 3.00 | 3.00 | 3.00 | 3.00 | 3.00 | 3.00 |
| Titanium Dioxide (and) Aluminum Stearate (and) Aluminum Hydroxide | 4.50 | 4.50 | 4.50 | 4.50 | 4.50 | 4.50 | 4.50 | 4.50 |
| Zinc Oxide | | | | | 4.00 | 4.00 | 4.00 | |
| Mineral Oil | 7.00 | 7.00 | 7.00 | 7.00 | 7.00 | 7.00 | 7.00 | 7.00 |

(continued)

| INCI Name | Ex.1 | Ex.2 | Comp Ex. 3 | Comp Ex. 4 | Ex.5 | Ex.6 | Comp Ex. 7 | Ex.8 |
|---|---|---|---|---|---|---|---|---|
| Capric/Caprylic Triglycerides | 3.75 | 3.75 | 3.75 | 3.75 | 3.75 | 3.75 | 3.75 | 3.75 |
| Cetyl Alcohol | | | 4.00 | 4.00 | | | 2.00 | |
| Hydroxyethylcellulose | | | 0.75 | 0.75 | | | 0.38 | |
| Laureth-4 | | | 1.56 | 1.56 | | | 1.56 | |
| Steareth-21 | | | 0.44 | 0.44 | | | 0.44 | |
| Sodium Methyl 2-Sulfolaurate Disodium 2-Sulfolaurate | | 0.65 | | 0.65 | | 0.10 | | |
| Methylparaben (and) Ethylparaben (and) Propylparaben (and) Butylparaben | 0.30 | 0.30 | | | 0.30 | | 0.30 | 0.30 |
| Methylparaben (and) Ethylparaben (and) Propylparaben | | | | | | 0.30 | | |
| Phenoxyethanol | | | | | | 1.00 | | |
| Methylparaben (and) Ethylparaben (and) Propylparaben (and) Butylparaben (and) Phenoxyethanol | | | 0.30 | 0.30 | | | | |
| Sodium Hydroxide | q.s. | q.s. | q.s. | q.s. | q.s. | q.s. | q.s. | q.s. |
| Citric Acid | q.s. | q.s. | q.s. | q.s. | q.s. | q.s. | q.s. | q.s. |
| Water | 80.70 | 80.05 | 74.70 | 74.05 | 76.70 | 75.05 | 73.07 | 79.40 |

**SPF Measurement**

The *in vitro* SPF scores were determined by using a Labsphere UV Transmittance Analyzer. The substrate used was an artifical skin testing substrate designed to mimic the topography, pH, critical surface tension, and ionic strength of human skin available commercially from IMS testing group under the tradename VitroSkin®, and the product was applied to the substrate at an application density of $2mg/cm^2$. Spectral scans of the transmission (or absorbance) were performed in the UV region of 250-450 nm. The *in vitro* SPF was then automatically calculated using the transmittance scan, along with solar spectral irradiance and CIE erythemal action spectra. At least 5 SPF values were recorded for each examples, and their averages are shown below in Table 2.

Table 2. Results of SPF Measurement:

**TABLE-2**

| Examples | SPF Value |
|---|---|
| Example 1 | 22.52 |
| Example 2 | 13.57 |
| Example 3 | 6.61 |

(continued)

| Examples | SPF Value |
|---|---|
| Example 4 | 6.12 |
| Example 5 | 26.71 |
| Example 6 | 24.77 |
| Example 7 | 9.31 |
| Example 8 | 15.69 |

EXAMPLES 9 and 10

The following two examples also serve as illustrations of the compositions of this invention, however, they do not limit the scope of the invention described herein.

Two personal care compositions, Examples 9 and 10 consistent with embodiments of the invention described herein, were prepared.

The emulsion systems shown in Examples 9 and 10 were prepared as follows: 3.0 parts glycerin were added to the amount of parts of water according to Table 1, less 15.0 parts water, solution agitated to uniform, 0.3 parts of parabens added and heated to 60-65°C until the parabens were dissolved. To this mixture 0.75 parts of ammonium acryloyldimethyltaurate/ VP copolymer were added and mixed with agitation until the solution is homogeneous, and the solution was then held between 60-65 °C for phasing (Phase 1 Solution). In a separate container, 7.0 parts of mineral oil and 3.75 parts capric/caprylic triglycerides, were heated to 60-65°C with agitation. In the case of Example 9, 4.5 parts titanium dioxide was added and mixed until dispersed. In the case of Example 10, 4.5 parts titanium dioxide and 2.0 parts zinc oxide were added and mixed until dispersed and then maintained between 60-65°C temperature. The solution was then added to the above solution (Phase 1 Solution) with rapid agitation. The heat was turned off, 15 parts water added to cool the batch, and agitation continued until the solution became homogenous. When the batch temperature reached 25-30°C, one part phenoxyethanol was added. In the case of Example 9, the pH was adjusted if necessary to be within the range of 5.5-6.5. In the case of Example 10, the batch was homogenized for 5 minutes at a 5 setting and the pH was adjusted if necessary to be within the range of 7.0-8.0.

**TABLE-3**

| INCI Name | Ex.9 | Ex.10 |
|---|---|---|
| Ammonium Acryloyldimethyltaurate/ VP Copolymer | 0,75 | 0.75 |
| Glycerin | 3.00 | 3.00 |
| Titanium Dioxide (and) Aluminum Stearate (and) Aluminum Hydroxide | 4.50 | 4.50 |
| Zinc Oxide | | 2.00 |
| Mineral Oil | 7.00 | 7.00 |
| Capric/Caprylic Triglycerides | 3.75 | 3.75 |
| Cetyl Alcohol | | |
| Methylparaben (and) Ethylparaben (and) Propylparaben (and) Butylparaben | 0.30 | 0.30 |
| Phenoxyethanol | 1.00 | 1.00 |
| Sodium Hydroxide | q.s. | q.s. |
| Citric Acid | q.s. | q.s. |
| Water | 80.70 | 80.05 |

**Rinse Resistance Measurement**

Rinse resistance was evaluated by determining the percentage of initial SPF after a 40 minute and 80 minute rinse cycle using a Labsphere UV Transmittance Analyzer. The substrate used was fresh chicken skin with dermis removed and rinsed in 96% ethanol. The product was applied to the skin by dispensing 4uL of product to a 16mm diameter skin sample and allowing product to dry for 15 minutes. Spectral scans of the transmission (or absorbance)

were performed in the UV region of 250-450 nm to obtain an initial *in vitro* SPF value. The skin sample was then immersed in a 19 liter water bath without agitation for 40 and 80 minutes time periods. After the desired time, the skin samples were removed from the bath an air dried for 20 minutes. After drying, the After Rinse *in vitro* SPF was then measured and percent of initial SPF calculated. The test was repeated three times to obtain a sample size of 4 for each test. Percentage of initial SPF was determined by dividing After Rinse SPF from Initial SPF and multiplying by 100. Results are recorded for Examples 9 and 10, and are shown below in Table 4.

Table 4. Results of Rinse Resistance Test:

**TABLE-4**

| **Examples** | **% of Initial SPF (%$R_{40}$) 40 Minutes** | **% of Initial SPF (%$R_{80}$) 80 Minutes** |
|---|---|---|
| Example 9 | 85 | 84 |
| Example 10 | 100 | 98 |

**Ocular Sting Test:**

Using a double-blinded, randomized, two (2) cell study test design, one (1) drop of a sample at a temperature of about 38°C is instilled into a subject's eye. A new sterile disposable eyedropper is used for each sample and disposed of after being used on only one individual's eye. All instillations are performed either by an investigator or by a trained technician.

Within 30 seconds, or as closely as possible following instillation, the subject is asked to grade the perceived stinging sensation to the eye utilizing the following criteria:

Sting

0 = Within normal limits
1 = Mild, very slight
2 = Moderate
3 = Severe

After 15 minutes and 60 minutes post-instillation, the subject is again asked to grade the perceived stinging sensation to the eye.

Results are recorded for Examples 9 and 10 , and are shown below in Table 5.

Table 5. Results of Human Ocular Sting Testing:

**TABLE-5**

| **Examples** | **% Ocular Sting Value*** |
|---|---|
| | (Example vs Control) |
| Example 9 | 10 |

(continued)

| Examples | % Ocular Sting Value* |
|---|---|
| | (Example vs Control) |
| Example 10 | 13 |

*With respect to ocular sting, the results of Table 5 were reported in terms of a weighted percentage of subjects who found the respective Example to be stinging to their eye versus those who perceived stinging when the control, i.e., sterile distilled water, was adminstered in their eye. In other words, the weighted percentage of subjects may be expressed in terms of:

$$\frac{X\,(100)}{(\text{total \# panelists})(\text{maximum intensity score})}$$

wherein X is the sum of [(#panelists responding for a given intensity criteria) (that intensity criteria chosen)]

As used herein, a composition is considered to be "mild to the eyes" if such composition results in an % Ocular Sting Value of less than 20%.

## Claims

1. An oil-in-water emulsion composition comprising an inorganic sunscreen and a polymeric emulsifier that is substantially free of hydrophobic modification, said emulsion composition being substantially free of organic sunscreens.

2. An oil-in-water emulsion composition comprising an inorganic sunscreen and a polymeric emulsifier that is substantially free of hydrophobic modification, said emulsion composition being substantially free of monomeric emulsifiers and mild to the eyes, and having an SPF value of at least about 15, an $\%R_{40}$ of at least about 90%, and an $\%R_{80}$ of at least about 90%.

3. An oil-in-water emulsion composition comprising an inorganic sunscreen comprising a combination of titanium dioxide and zinc oxide, and a polymeric emulsifier that is substantially free of hydrophobic modification.

4. The emulsion composition of claim 1 having an SPF value of at least about 15.

5. The emulsion composition of claim 1 or claim 2 having an SPF value of at least about 20.

6. The emulsion composition of claim 1 having a $\%R_{40}$ of at least about 90%.

7. The emulsion composition of claim 1 or claim 2 having a $\%R_{40}$ of at least about 95%.

8. The emulsion composition of claim 1 having a $\%R_{80}$ of at least about 90%.

9. The emulsion composition of claim 1 or claim 2 having a $\%R_{80}$ of at least about 95%.

10. The emulsion composition of claim 1 wherein said composition is mild to the eyes.

11. The emulsion composition of claim 1 wherein said inorganic sunscreen agent is selected from the group consisting of metal oxides, silica, aluminosilicates, carbonates, and combinations of two or more thereof.

12. The emulsion composition of claim 11 wherein said inorganic sunscreen agent is selected from the group consisting of titanium dioxide, zinc oxide, and combinations thereof.

13. The emulsion composition of claim 12 wherein said inorganic sunscreen agent comprises a combination of titanium dioxide and zinc oxide.

**14.** The emulsion composition of claim 1 or claim 3 wherein said polymeric emulsifier is defined by the formula:

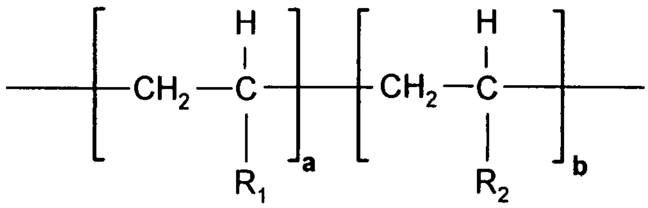

wherein,
a is from about 0 to 0.99, b is from about 0.01 to 1, where a+b=1 and $R_1$ and $R_2$ are either identical or different moieties defined by the formula $X\text{-}Y\text{-}Z^- M^+$, wherein X is a moiety selected from the group consisting of -COO-, -OCO-, -CONH-, and -NHCO-; Y is a linear, branched, or cyclic ($C_{1\text{-}4}$) alkyl group; Z is an anionic functional group selected from the group consisting of $-COO^-$, $-SO_3^-$, $-SO_4^-$, $-PO_3^-$ and $-PO_4^-$; and $M^+$ is selected from the group consisting of $Li^+$, $Na^+$, $K^+$, and $NH_4^+$.

**15.** The emulsion composition of claim 1 or claim 3 wherein said polymeric emulsifier is a copolymer of acrylamidoalkyl sulfonic acid and cyclic N-vinylcarboxamides.

**16.** The emulsion composition of claim 1 wherein said polymeric emulsifier is an AMPS homopolymer.

**17.** The emulsion composition of claim 2 wherein said inorganic sunscreen agent is selected from the group consisting of titanium dioxide, zinc oxide, and combinations thereof, and said polymeric emulsifier is selected from the group consisting of copolymers of acrylamidoalkyl sulfonic acid and cyclic N-vinylcarboxamides, AMPS homopolymers, and combinations of two or more thereof.

**18.** The emulsion composition of claim 3 wherein said composition is substantially free of monomeric emulsifiers.

**19.** A cosmetic method of protecting the skin from photodamage comprising applying a composition of claim 1, claim 2 or claim 3 to the skin.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5980871 A
- US 6540986 B2, Lukenbach
- US 20040067208 A1, Lennon
- US 20040071641 A1, Boutelet